# EUROPEAN PATENT APPLICATION

(11) **EP 1 650 329 A2**
(43) Date of publication of application: **26.04.2006**
(21) Application number: 05256541.3
(22) Date of filing: 21.10.2005
(51) Int. Cl.: C25F 1/00

(54) **Apparatus and method for electrolytic cleaning**

(30) Priority: 21.10.2004 GB 0423339; 27.10.2004 GB 0423817
(71) Applicant: Trust Sterile Services Limited, Bellshill, Lanarkshire ML4 3NJ (GB)
(72) Inventor: Prior, Frank c/o Trust Sterile Services Ltd., Bellshill Lanarkshire, ML4 3NJ (GB)
(74) Representative: Ede, Eric

(57) **Abstract**

The present invention relates to apparatus and methods that can be used to clean instruments, in particular by removing proteins from surgical instruments. The apparatus comprises an electrolytic device comprising an anode (6), a cathode (11), an electrolyte (12) and a power supply (3), wherein the cathode is an instrument to be cleaned. The method relates to the use of said apparatus for cleaning instruments.

## Description

The present invention relates to the field of electrolytic cleaning devices. The invention also relates to an improved method of cleaning by means of an electrolytic device. In particular, the apparatus and method relate to an electrolytic device for cleaning surgical instruments.

The efficient cleaning of instruments and, in particular, surgical instruments, has become of increasing importance due to the prevalence of blood borne diseases, such as new variant Creutzfeldt-Jakob disease (CJD - so-called "mad cow" disease). Prion proteins are the suspected causative agent of new variant CJD and are recognised as one of the most difficult brain proteins to remove from surgical instruments using conventional cleaning apparatus and methods. The effective cleaning of surgical instruments is of particular importance when the instruments are non-disposable, and are to be used on different patients in different operations. This problem is exacerbated due to the fact that the proteins in blood and tissues (such as brain tissue), bind firmly onto stainless steel, which is often used to manufacture surgical instruments. Various apparatus and methods for cleaning surgical instruments exist but none of these addresses the issue of completely removing blood or tissue proteins or tissues from the surface of the instruments. In fact, several existing methods employ techniques that exacerbate the problem of blood and tissue protein adhering to the stainless steel surfaces of surgical instruments.

Contact of brain or blood materials with any (even slightly) acidic substance, such as an alcohol, renders the proteins contained therein insoluble. This makes the removal of tissue and blood materials and the proteins contained therein, even more difficult (see F G R Prior et al, *Journals of Hospital Infection,* **2004,** 58, 78-80). Similar problems have been reported with the use of formaldehyde and paraldehyde - they also render the proteins in blood and tissue insoluble, and make them harder to remove from surgical instruments. As alcohol is used frequently and in large quantities in hospitals (and in particular operating theatres) as a skin and surface disinfectant, it is inevitable that the alcohol will come into contact with soiled surgical instruments, thereby exacerbating the problems associated with the cleaning of said instruments.

Electrolytic devices for cleaning surgical instruments are known to the art. In particular, blood can be removed from surgical instruments by soaking in alkaline solutions with a pH of about 12. However, brain material is particularly resistant to cleaning, and often requires further treatment (Bernoulli C, Siegfried J, Baumgartner G et al, Danger of accidental person to person transmission of Creutzfeldt-Jacob disease by surgery Lancet 1977; 1: 478-479). Often, soaking in hot concentrated sodium hydroxide or concentrated sodium hypochlorite is required to remove brain tissue (Taylor DM, Inactivation of transmissible degenerative encephalopathy agents: a review Vet J, 2000; 159: 3-4). Even so, such treatments do not completely remove some proteins, and in particular they do not remove prion proteins (Taylor DM, Fernie K, McConnell I, Steel PJ, Survival of scrapie agent after exposure to sodium dodecylsulphate and heat Vet Microbiol 1999; 1: 13-16).

Existing washer dryer devices for the cleaning of blood stained materials require a delicate balance to be found between the correct pH, the correct type and amount of detergent, the amount of ultrasonic vibration and in certain machines the correct type and amount of enzyme. This balance can be difficult to achieve, and therefore can prove time consuming. More importantly, such washer dryer cleaning devices do not remove all of the protein and, in particular, prion proteins, that become attached to instruments.

Solvent based cleaning processes for surgical instruments need to be able to remove alcohol bound blood and tissue (such as brain tissue) from the surgical instrument surfaces. This is particularly important in the knowledge that the proteins in blood and tissues, such as brain tissue, bind firmly onto stainless steel. In particular, prion proteins, the suspected causative agent of new variant CJD (mad cow disease) are recognised as one of the most difficult brain proteins to remove from surgical instruments using conventional cleaning apparatus and methods.

The only effective solvent cleaning method that has been demonstrated to date capable of removing transmissible spongiform encephalopathy (TSE) infectivity is prolonged exposure to hot concentrated sodium hydroxide (Taylor DM, Inactivation of transmissible degenerative encephalopathy agents: a review Vet J, 2000; 159: 3-4). Other solvent methods do not completely remove blood and tissue proteins and, in particular, prion proteins, from soiled surgical instruments. In addition hot concentrated sodium hydroxide is extremely caustic.

Several inventions describe the use of electrolytic devices for cleaning devices. These are based on either electrolysing dilute solutions of sodium chloride to produce sodium hypochlorite, US patent number US 4402197, or the electrolysis of water to produce H⁺ enriched acid water at the anode water interface and alkaline HO⁻ enriched water at the cathode water interface. The following documents describe devices for electrolysing water. US 3616355, US 4048047, US 4062752, US 4100052,

US 4328084, US 4761208, US 5314589, US 5395492, US 5439576, US 5954939 (EP 711730) and (WO 00/34184).

Document US 5534120 describes an attached, non partitioned electrochemical cell, which can optimally separate the acidic/alkaline ionized water streams separately in dishwashers. Document US 5947135 describes the use of an attached partitioned electrochemical cell that produces separate analyte/cathodic streams for cleaning and disinfection of tableware. JP 10033448 describes the use of an attached electrochemical cell in conjunction with an alkaline cleaning agent containing enzymes to clean tableware.

In all of the present electrolytic methods the instrument to be cleaned is placed in the electrolytic bath, between the separate anode and cathode.

One problem encountered with these devices is fouling of the electrochemical cells with scaling. Several remedies have been proposed. JP 10057297 and US 5954939 reduce scaling by electrode polarity reversal; WO 00/64325 and US 4434629 incorporate the electrochemical cell as part of a water softening system to reduce scaling. US 5932171 incorporates the use of an acid or other descaler to purge the electrochemical cell.

As described above, contact with acid makes blood more difficult to remove from stainless steel by making it insoluble in water. The acidified water produced by the above inventions make the cleaning of surgical instruments more difficult. Electrolytic devices that produce chlorine or other halide gasses have to be used cautiously as the halides are extremely poisonous. Hot strong alkalinic solutions are effective in destroying prion protein, but none of these electrical devices can produce alkali strong enough to inactivate CJD infectivity.

Present electrolytic methods do not produce complete cleaning of surgical instruments, and they are generally time consuming and inefficient. It would therefore be desirable to devise a cleaning apparatus and method that obviates the use of one or more of these components, such that it is quicker and easier to develop an efficient cleaning mixture and mechanism.

It would therefore be desirable to obviate or at least mitigate some of the drawbacks associated with the prior art.

It is therefore an object of the present invention to provide an apparatus for electrolytic cleaning.

It is a further object of the present invention to provide an electrolytic device for the removal of blood and tissue from instruments.

A still further aim of the invention is to provide an electrolytic device for the removal of proteins, in particular prion proteins from instruments.

A further aim of the invention is to provide a method of electrolytic cleaning.

A still further aim of the invention is to provide a method for the sterilisation of surgical instruments.

A still further object of the invention is to provide a method for the removal of proteins and, in particular, prion proteins, from instruments.

In this description, instruments and instrumentation are not intended to be limited to surgical tools, but relate to any device that it may conceivably be desirable to remove blood or tissue, and especially blood or tissue proteins, from. For example, instruments or instrumentation may encompass such devices used in industries such as medicine, food preparation, veterinary medicine, tattooing, ear piercing, scientific research and laboratory use.

The aforesaid aims are achievable by the present invention which provides an electrolytic method and device wherein the cathode comprises the instrument to be cleaned.

According to a first aspect of the present invention, there is provided an electrolytic cleaning device comprising:
- an anode;
- a cathode;
- an electrolyte; and
- a means for supplying power;
wherein the cathode is an instrument which is to be cleaned.

Preferably the electrolytic cleaning device comprises an electrolytic bath that contains the electrolyte.

Preferably the anode and the cathode are immersed in the electrolyte.

Preferably the anode and the cathode are connected to the means for supplying power by anode- and cathode-connecting wires respectively.

Optionally the cathode-connecting wire is attached to a connecting device.

Preferably the instrument to be cleaned is attached to a connecting device.

Preferably the electrolytic cleaning device comprises a means for switching on the power supply.

Preferably the electrolytic cleaning device comprises a means for switching on the power supply which is interposed between the means for supplying power and one or both of the anode and cathode.

Optionally the means for switching on the power supply is situated elsewhere in the electrolytic cleaning device.

Preferably the instrument is a surgical instrument.

Optionally the surgical instrument is fabricated from stainless steel.

Preferably the anode comprises the same material as the cathode.

Optionally the anode comprises a material that is different from the cathode.

Preferably the anode is sacrificial.

Preferably the cathode and anode are situated approximately one inch (2.54 cm) apart.

Preferably the electrolyte comprises a salt solution.

Preferably the electrolyte is alkaline.

Preferably the electrolyte comprises an alkali metal.

Preferably the electrolyte comprises sodium carbonate.

More preferably the electrolyte comprises 0.3 to 1.5% sodium carbonate (Na₂CO₃).

Optionally the electrolyte comprises sodium bicarbonate (NaHCO₃).

Preferably on operation of the apparatus, the chosen electrolyte will produce one or more gases, which offers an advantage in that one may monitor the electrolytic process with regard to determining the pattern of bubbles produced. In practice it is found there is a simple size distinction between bubbles issuing from contaminated instrument surfaces and those issuing from clean surfaces thereby providing an indicator for achieving a clean instrument. Thus a comparison can be made and the power switched off when there is a change in the bubble pattern that indicates that the instrument is clean.

According to a second aspect of the present invention, there is provided a generic method of cleaning an instrument, comprising the steps of:
- connecting the instrument to the negative terminal of a power supply;
- immersing the instrument in an electrolyte;
- bringing an anode into contact with the electrolyte;
- switching on the power supply;
wherein the instrument attached to the negative terminal of the power supply forms the cathode.

Preferably, the anode is substantially submerged in the electrolyte.

Preferably the instrument is a surgical instrument.

Optionally the surgical instrument is fabricated from stainless steel.

Preferably the anode comprises the same material as the cathode.

Optionally the anode comprises a material that is different from the cathode.

A further step is the removal and disposal of the anode once the electrolytic cleaning is completed.

Preferably the cathode and anode are situated approximately one inch (2.54 cm) apart.

Preferably the electrolyte comprises a salt solution.

Preferably the electrolyte is alkaline.

Preferably the electrolyte comprises sodium carbonate.

More preferably the electrolyte comprises 0.3 to 1.5% sodium carbonate (Na₂CO₃).

Optionally the electrolyte comprises sodium bicarbonate (NaHCO₃).

Preferably the power supply is maintained at substantially 12 volts.

Preferably the apparatus is operated for approximately 30 minutes.

Preferably on carrying out the method, the chosen electrolyte will produce one or more gases.

Preferably the gases produced create bubbles on one or both of the anode and cathode.

More preferably the apparatus is viewed and is switched off when a there is a change in the bubble pattern that indicates that the instrument is clean.

Alternatively, the step of monitoring the electrolytic process to determine the pattern of bubbles produced, may be carried out by an instrumental technique utilising detection means adapted to switch off the power when there is a change in the bubble pattern that indicates that the instrument is clean.

Various embodiments of the present invention will now be described by way of example only, with reference to the accompanying single Figure, of which:

The sole Figure is a two-dimensional schematic diagram of the apparatus for electrolytic cleaning.

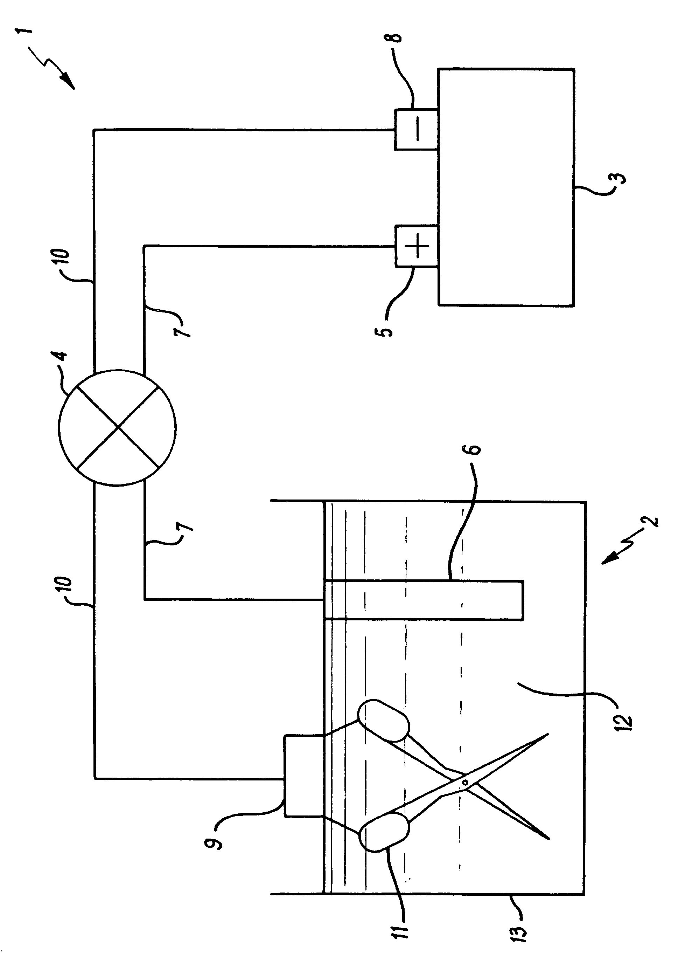

Referring to Figure 1, there is depicted an apparatus for electrolytic cleaning **1**, comprising an electrolytic cell **2**, a power supply **3** and a switch **4**. The positive terminal **5** of the power supply 3 is connected to an anode **6** by means of an anode-connecting wire **7**. The negative terminal **8** of the power supply 3 is connected to the connecting device **9** by means of the cathode-connecting wire **10**. The connecting device 9 attaches to the instrument **11**, thereby forming the cathode **11**.

The connecting device may take the form of a crocodile clip. Alternatively, it may take the form of a wire loop.

The instrument/cathode 11 and the anode 6 are immersed in an electrolyte solution **12**, all of which is contained in an electrolytic bath **13**. In this example, the electrolyte solution 12 is 0.3% sodium carbonate solution, however, any suitable electrolyte solution can be used. For example, sodium bicarbonate solution would also be a suitable electrolyte. In particular, it is desirable to use an electrolyte that, when subjected to electrolysis, will generate one or more gases. The anode 6 need not necessarily be immersed in the electrolyte solution 12 but could, for example, also form the electrolytic bath 13.

The instrument/cathode 11 in this example is a set of surgical scissors fabricated from stainless steel. To complement the instrument/cathode 11, the anode 6 in this example is also fabricated from stainless steel. The anode is electrolytically decomposed during the process while the instrument/cathode remains free of damage. Therefore, the anode 6 in this particular example is sacrificial and may be discarded at the end of use. In this example, the anode 6 and the cathode 11 are located approximately one inch (2.54 cm) apart and the electrolyte solution is 0.3% sodium carbonate solution. It will be appreciated that although this particular example refers to stainless steel, any suitable conductor may be attached to the connecting device 9, in order to form a cathode 11. It will also be appreciated that any suitable anode 6 may be used, such that it complements the cathode 11, and allows the formation of a working cell.

In use, the instrument 11 is connected to the connecting device 9 and electrolyte solution 12 is added to the electrolytic bath 13, such that the instrument 11 is totally immersed. The anode 6 is then submerged in the electrolyte solution 12 contained in the electrolytic bath 13. The power supply 3 is then switched on by means of the switch 4.

When the power supply 6 is switched on, both the anode 6 and the cathode 11 start to bubble, indicating (in the case of a sodium carbonate or sodium bicarbonate electrolyte) the generation of carbon dioxide and hydrogen respectively. The soiled area (not shown) of the instrument/cathode 11 becomes covered with a white mist of small bubbles (not shown). Non-contaminated areas produce much larger bubbles. As the cleaning progresses, the soiled area (not shown) containing protein (not shown) is removed and the bubble pattern reverts to a surface of large bubble production. After approximately 30 minutes, the small bubbles disappear indicating that the instrument 11 is clean, and that the fixed protein has been removed from the instrument 11. The instrument 11 may then be detached from the connecting device 9 and is in a suitable condition to be put through a normal washer dryer process. In this example, the anode 6 is sacrificial and can be removed and destroyed after use, and the power supply 3 is a 12 Volt battery.

The present invention utilises the fact that proteins carry electrical charge. If the metal instrument that requires cleaning is suspended in an alkaline solvent and connected to an electrical circuit so that the polarity of the charged instrument is the same as that of the protein, the protein will be repelled from the instrument into the body of the solvent and eventually onto the alternative electrode.

Blood and brain proteins, and in particular prion proteins, have an overall negative charge. Therefore, when the instrument is employed as a cathode and a negative charge is applied, the negatively charged attached proteins are repelled from the instrument thereby sterilizing it.

Slow soaking of the blood and tissue, or soil, attached to the instrument in the electrolyte solution, and the electrical repulsion between the protein and the cathode, and the physical activity of the gas bubbles on the instrument surface, combine to remove the protein from the instrument. Initial results, shown on Table 1, illustrate the effectiveness of the apparatus and method in removing soil from surgical instruments.

**Table 1 Initial results for the removal of soil from stainless steel using the electrolytic cleaning device and method, and using a 12 Volt battery as the power supply.**

| Soil On Stainless Steel | Time until clean (min) 0.3 % Na₂CO₃ | Time until clean (min) 0.6 % Na₂CO₃ |
|---|---|---|
| blood | 4 | - |
| blood + alcohol | 10 | - |
| brain | 10, 12 | 5, 10 |
| brain + alcohol | 20, 20, 25 | 14, 18, 20, 20 |

It should be noted that the apparatus and method for electrolytic cleaning may have several applications in a variety of technologies, some of which have not been mentioned explicitly herein. In particular, the apparatus and method for electrolytic cleaning will be useful in the fields of surgery, medicine, food preparation, veterinary medicine, tattooing, ear piercing, scientific research and laboratory use.

The arrangement shown in the Figure is exemplary only, and it will be apparent that other arrangements of the apparatus and method for electrolytic cleaning can exist. Therefore, the specific structural and functional details disclosed herein are not to be interpreted as limiting, but merely as the basis for the Claims and for teaching one skilled in the art as to the various uses of the present invention in any appropriate manner.

## Claims

1. An electrolytic cleaning device comprising an anode (6), a cathode (11), an electrolyte (12) and a means for supplying power (3) for the electrolytic cleaning, ***characterised in that*** the cathode comprises an instrument which is to be cleaned.

2. An electrolytic cleaning device according to Claim 1, ***characterised in that*** the device comprises an electrolytic bath (13) that contains the electrolyte.

3. An electrolytic cleaning device according to either of the previous Claims, ***characterised in that*** the anode and the cathode are immersed in the electrolyte.

4. An electrolytic cleaning device according to any one of the preceding Claims, ***characterised in that*** the anode and the cathode are connected to the means for supplying power by anode- and cathode-connecting wires respectively (7,10).

5. An electrolytic cleaning device according to Claim 4, ***characterised in that*** the cathode-connecting wire (10) is attached to a connecting device (9).

6. An electrolytic cleaning device according to Claim 5, ***characterised in that*** the instrument to be cleaned is attached to the connecting device.

7. An electrolytic cleaning device according to any one of the preceding Claims, ***characterised in that*** the electrolytic cleaning device comprises a means (4) for switching on the power supply (3).

8. An electrolytic cleaning device according to Claim 7, ***characterised in that*** the means for switching on the power supply is interposed between the means for supplying power and one or both of the anode and cathode.

9. An electrolytic cleaning device according to any one of the preceding Claims, ***characterised in that*** the anode comprises the same material as the cathode.

10. An electrolytic cleaning device according to any of Claims 1 to 8, ***characterised in that*** the anode comprises a material that is different from the cathode.

11. An electrolytic cleaning device according to any one of the preceding Claims, ***characterised in that*** the anode is sacrificial.

12. An electrolytic cleaning device according to any one of the preceding Claims, ***characterised in that*** the electrolyte is alkaline.

13. An electrolytic cleaning device according to Claim 12, ***characterised in that*** the electrolyte comprises an alkali metal.

14. An electrolytic cleaning device according to any one of the preceding Claims, ***characterised in that*** the electrolyte comprises a salt solution.

15. An electrolytic cleaning device according to Claim 14, ***characterised in that*** the electrolyte comprises at least one of sodium carbonate (Na₂CO₃) and sodium bicarbonate (NaHCO₃).

16. An electrolytic cleaning device according to any one of the preceding Claims, ***characterised in that*** on operation of the device for cleaning of an instrument, the electrolyte will produce one or more gases.

17. A method of cleaning an instrument, ***characterised by*** the steps of:
• connecting the instrument to the negative terminal of a power supply (3);
• immersing the instrument in an electrolyte (12);
• bringing an anode (6) into contact with the electrolyte (12);
• switching on the power supply (3);
wherein the instrument attached to the negative terminal of the power supply (3) forms a cathode (11).

18. A method according to Claim 17, in which the anode is submerged in the electrolyte.

19. A method according to either Claim 17 or Claim 18, ***characterised in that*** the method further includes the step of the removal and disposal of the anode (6) once the electrolytic cleaning is completed.

20. A method according to any of Claims 17 to 19, ***characterised by*** the step of monitoring the electrolytic process to determine the pattern of bubbles produced, comparing the pattern with a predetermined pattern that is indicative of a clean instrument, and switching the power off when there is a change in the bubble pattern that indicates that the instrument is clean.

21. A method according to claim 20, wherein the step of monitoring the apparatus to determine the pattern of bubbles produced is carried out by viewing said pattern.

22. A method according to claim 20, wherein the step of monitoring the apparatus to determine the pattern of bubbles produced is carried out by an instrumental technique, utilising detection means adapted to switch off the power.
